# EUROPÄISCHE PATENTSCHRIFT

(11) **EP 1 135 366 B1**
(45) Veröffentlichungstag und Bekanntmachung des Hinweises auf die Patenterteilung: **01.10.2003**
(21) Anmeldenummer: 99958128.3
(22) Anmeldetag: 26.11.1999
(51) Int. Cl.: C07C 273/12

(54) **VERFAHREN ZUR EINBINDUNG DER MELAMIN-OFFGASE IN EINE HARNSTOFFANLAGE**
METHOD OF INTRODUCING MELAMINE OFF-GASES INTO A UREA PLANT
PROCEDE POUR INTRODUIRE PAR LIAISON DES EFFLUENTS GAZEUX DE MELANINE DANS UNE INSTALLATION POUR PRODUIRE DE L'UREE

(30) Priorität: 03.12.1998 AT 203998
(43) Veröffentlichungstag der Anmeldung: 26.09.2001
(73) Patentinhaber: Agrolinz Melamin GmbH, A-4021 Linz (AT)
(72) Erfinder: COUFAL, Gerhard, A-4060 Leonding (AT)
(74) Vertreter: Gross, Felix
(86) Internationale Anmeldenummer: EP9909192
(87) Internationale Veröffentlichungsnummer: WO00032566

(56) Entgegenhaltungen:
- WO-A-98/08808
- WO-A-98/32731

## Beschreibung

Die Erfindung betrifft die Einbindung der Melamin- Offgase in eine Harnstoffanlage mittels Ejektoren.

Die bei der Melaminsynthese anfallenden, insbesondere aus NH₃ und CO₂ bestehenden Offgase, werden üblicherweise zur Herstellung von Harnstoff eingesetzt. Dabei werden vorteilhafterweise die Offgase aus der Melaminanlage direkt in die Harn-stoffanlage übergeführt, wo sie beispielsweise in einem Carbamatstrom absorbiert und in den Reaktor weitergefördert werden. Eine verbesserte, rationellere und wirtschaftlichere Methode der Offgaseinbindung wird in K. Abe et al.-Kagaku Kogaku 40, 298-302 (1976) beschrieben, bei der die Offgase, gegebenenfalls nach Entfernen von restlichem Melamin in einem Harnstoffwäscher, direkt und unverändert in trockenem Zustand in den Hochdruckteil der Harnstoffanlage eingebracht werden. Diese Verfahrensvariante ist auch in SU 899538 oder WO 98/08808 beschrieben. Gemäß WO 98/32731 werden die Melamin- Offgase vorerst beim Druck des Melaminreaktors kondensiert, wobei Ammoniumcarbamat entsteht, das anschließend in den Hochdruckteil der Harnstoffanlage gefördert wird.

Der Nachteil der bekannten Verfahren besteht vor allem darin, daß zum Teil Energieverluste durch Entspannen bzw. Abkühlen der Offgase auftreten, mit den dadurch bedingten zusätzlichen Verfahrensschritten und Apparateteilen, bzw. daß im Falle der Direktüberführung der Offgase in die Hamstoffanlage der Druck der Offgase bzw. der Druck im Melaminreaktor größer sein muß als der Druck im Harnstoffreaktor. Dies bedingt eine unflexible und starr vorgegebene Fahrweise der beiden Reaktoren, wodurch die Reaktoren oft nicht unter den für den jeweiligen Prozeß optimalen Bedingungen gefahren werden können.

Es wurde nun unerwarteterweise gefunden, daß diese Nachteile dann ausgeschaltet werden können, wenn die Melamin- Offgase mittels Ejektoren direkt in den Hochdruckteil der Harnstoffanlage eingebracht werden.

Gegenstand der Erfindung ist demnach ein Verfahren zur Herstellung von Harnstoff, bei dem die aus einer Melaminanlage stammenden, im wesentlichen aus NH₃ und CO₂ bestehenden Gase (Melamin-Offgase), mittels eines oder mehrerer Ejektoren direkt in den Hochdruckteil einer Harnstoffanlage eingebracht werden. Zum Hochdruckteil der Harnstoffanlage zählen insbesondere der Reaktor, der Stripper und Carbamatkondensor, sowie in diesem Anlagenbereich liegende Leitungen und Apparateteile.

Das vorliegende Verfahren eignet sich für beliebige Melaminanlagen und Harnstoffanlagen. Solche Anlagen sind beispielsweise aus "Ullmann's Encyclopedia of Industrial Chemistry, 5. ed, vol. A16 (1990) Seiten 171-185 (Melamin) bzw. vol. A27 (1996) Seiten 333-365 (Harnstoff), sowie aus K. Abe et al, Kagaku Kogaku 40 (1976) Seiten 298-302, aus EP-727,414 A, WO 98/08808 und WO 98/32731 bekannt.

Die im vorliegenden Verfahren verwendeten Melamin- Offgase stammen bevorzugt aus einer Melamin-Hochdruckanlage, in der Melamin bei Temperaturen von 300 - 500 °C und Drücken von 80 - 800 bar aus Harnstoff unter Abspaltung von NH₃ und CO₂ erhalten wird. Die abgespaltenen, im wesentlichen NH₃ und CO₂ enthaltenden Offgase werden erfindungsgemäß direkt in eine Harnstoffanlage geleitet, bevorzugt nach Durchleiten durch geschmolzenen Harnstoff (Harnstoffwäscher) zur Entfernung von restlichem Melamin, wie beispielsweise in Ullmann beschrieben. Besonders geeignete Harnstoffanlagen sind solche, welche auf dem " Harnstoff-stripping process" basieren, wobei die Gase bei 150 bis 350 °C, bevorzugt bei 170 bis 200 °C und 125 bis 350 bar, bevorzugt bei 140 bis 200 bar, zu Harnstoff umgesetzt werden. Nicht zu Harnstoff umgesetztes NH₃ und CO₂ wird in einem anschließenden Stripper ausgetrieben und anschließend in einem Kondensor kondensiert, wobei sich NH₃- und CO₂ -hältiges NH₄ -Carbamat bildet, das wieder in den Harnstoffreaktor rückgeführt wird. Die aus dem Stripper austretende Harnstofflösung wird in anschließenden weiteren Zersetzern bei fallenden Drücken, beispielsweise in einem Mitteldruckzersetzer, anschließend in einem Niederdruckzersetzer, sowie Vakuumverdampfer, durch weitere Zersetzung der enthaltenen Carbamate und Carbonate sowie Austreiben von NH₃ und CO₂ aufkonzentriert. Die bei der Aufkonzentrierung anfallenden Gase werden kondensiert und anschließend in den Harnstoffprozeß zurückgeführt.

In einem bevorzugten Verfahren gemäß vorliegender Erfindung werden die Ejektoren zur Förderung der Melamin- Offgase in den Hochdruckteil der Harnstoffanlage mit einem oder mehreren der folgenden Ströme als Treibmedien betrieben:
a) flüssiges NH₃,
b) gasförmiges NH₃ oder CO₂,
c) im wesentlichen NH₃ und CO₂ enthaltende wäßrige Lösungen.

Die im wesentlichen NH₃ und CO₂ enthaltenden wäßrigen Lösungen fallen bevorzugt in einer Harnstoffanlage an, sie können jedoch auch bei anderen Prozessen, bei denen NH₃ und CO₂ anfallen stammen, beispielsweise aus einer Melaminanlage. Die bei der Herstellung des Harnstoffs, insbesondere aus der Aufarbeitung stammenden, im wesentlichen NH₃ und CO₂ enthaltenden wäßrigen Lösungen, die beispielsweise aus dem Carbamat-Kondensor oder aus dem Niederdruckteil der Harnstoffanlage, beispieweise aus dem Mitteldruckabsorber stammen, können erfindungsgemäß als Treibmedium für die Ejektoren zur Einbringung der Melamin-Offgase in den Hochdruckteil der Harnstoffanlage verwendet werden.

Ein besonderer Vorteil der Verwendung der aus dem Sumpf des Mitteldruckabsorbers stammenden, neben NH₃, CO₂, H₂O und Carbamaten auch Carbonate enthaltenden Lösungen, besteht insbesondere darin, daß sie einen niederen Ausgasedruck der gelösten Gase aufweisen. Es erweist sich nämlich als günstig, wenn der Ausgasedruck der Treibmittel, gegebenenfalls auch die Temperatur, niedriger liegen als der Druck und gegebenenfalls die Temperatur der einzubringenden Melamin-Offgase. Dadurch wird verhindert, daß gelöste Gase im Ejektor ausgasen.

Der Druck der verwendeten Treibmedien liegt je nach Art und Menge des Treibmediums so, daß entsprechend den Ansaugbedingungen und der Menge der zu fördernden Offgase, sowie dem jeweiligen Gegendruck im Hochdruckteil der Harnstoffanlage möglichst die gesamte anfallende Menge an Offgasen gefördert werden kann. Der Druck und die Menge der Treibmedien muß demnach entsprechend hoch sein, um zu gewährleisten, daß die zu fördernden Offgase in der jeweiligen Menge beim jeweiligen Druck in den Hochdruckteil der Harnstoffanlage eingebracht werden können. Der Druck der Treibmedien ist dabei höher als der Druck im Hochdruckteil der Harnstoffanlage und ist bevorzugt 1,1 bis 3 mal so hoch, besonders bevorzugt 1,3 bis 2,5 mal so hoch wie der Druck im Hochdruckteil der Harnstoffanlage. Die Temperatur der Treibmedien ist vor allem von der jeweiligen Prozeßführung abhängig und liegt bevorzugt in einem Bereich von 10 °C bis 200 °C. Im Falle der Verwendung von NH₃, beispielsweise des Synthese- NH₃ für die Harnstoffherstellung erweist sich eine Temperatur von 10 °C bis 80 °C, besonders bevorzugt von 20 °C bis 65 °C als vorteilhaft. Im Falle der Verwendung von Synthese-CO₂ liegt die Temperatur bevorzugt etwas höher, bei 115 °C bis 140 °C. NH₃ und CO₂ enthaltende wäßrige Lösungen als Treibmittel, die beispielsweise aus dem Carbamatkondensor der Harnstoffanlage stammen, wobei gleichzeitig beispielsweise 3 bis 4 bar Dampf erzeugt wird, besitzen bevorzugt Temperaturen von 150 °C bis 160 °C. Rückgeführte Carbonatlösungen. beispielsweise aus dem Mitteldruckabsorber der Harnstoffanlage können Temperaturen von 65 °C bis 100 °C, bevorzugt von 65 °C bis 70 °C besitzen.

Das Mol-Verhältnis von NH₃ zu CO₂ in den einzubringenden, aus der Melaminanlage stammenden Melamin-Offgasen hängt von der Art des verwendeten Melaminprozesses ab und liegt bevorzugt bei 2,5 bis 5. Der Druck der aus der Melaminanlage stammenden Melamin- Offgase entspricht im wesentlichen dem Druck im Melaminreaktor und liegt bevorzugt bei 50 bis 250 bar, besonders bevorzugt bei 70 bis 200 bar. Die Temperatur der Melamin- Offgase liegt bevorzugt bei 175 bis 250 °C, besonders bevorzugt bei 180 bis 210 °C.

Der besondere Vorteil des erfindungsgemäßen Verfahrens liegt demnach insbesondere in der Verwendung der rückzuführenden Produktströme als Ejektor-Treibmedien, sowie auch in der Verwendung der als Einsatzstoffe verwendeten NH₃ und CO₂ als Ejektor-Treibmedien. Dadurch wird einerseits eine ökonomische Fahrweise erreicht, und andererseits auch die Möglichkeit gegeben, die Harnstoff- und Melaminproduktion flexibel und unter jeweils optimalen Bedingungen zu fahren, wobei auch niedrigere Melamin-Reaktordrücke als Harnstoff- Reaktordrücke möglich sind.

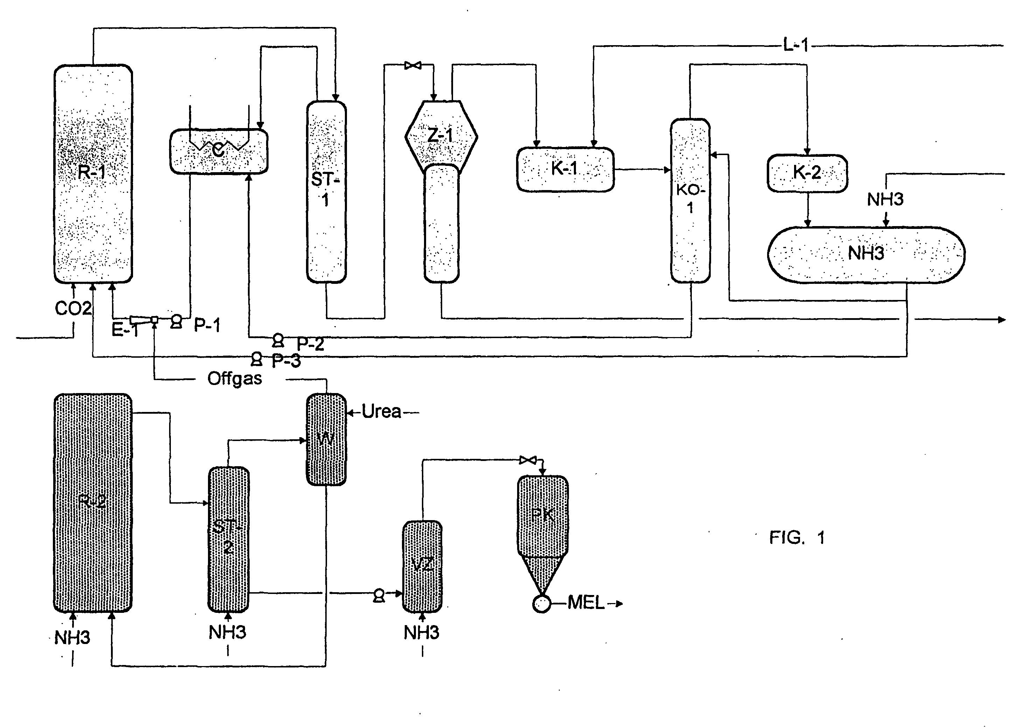

In Fig. 1 bis Fig. 4 sind beispielhaft 4 mögliche erfindungsgemäße Varianten der Einbringung der Melamin-Offgase in den Harnstoffreaktor mit Hilfe von Ejektoren abgebildet. In Fig. 1 bis Fig. 4 sind die folgenden wesentlichen Anlageteile der Harnstoffanlage und der Melaminanlage dargestellt:

| Harnstoffanlage: | |
|---|---|
| R-1 | Harnstoffreaktor |
| C | Carbamat-Kondensor |
| ST-1 | Harnstoffstripper |
| Z-1 | Zersetzer/Separator |
| K-1 | Kondensator 1 |
| K-2 | Kondensator 2 |
| K 0-1 | Mitteldruckabsorber |
| E-1 | Ejektor 1 |
| E-2 | Ejektor 2 |
| E-3 | Ejektor 3 |
| P-1 | Carbamat-Pumpe |
| P-2 | Carbonat-Pumpe |
| P-3 | NH₃-Pumpe |

| Melaminanlage | |
|---|---|
| R-2 | Melaminreaktor |
| ST-2 | Melaminstripper |
| W | Harnstoffwäscher |
| VZ | Verweilzeitbehälter |
| PK | Produktkühler |

### Beispiel 1

In einer Analge, wie sie schematisch in Fig. 1 dargestellt ist, werden vom Kopf des Harnstoffreaktors R-1 die Reaktionsprodukte zu einem dampfbeheizten Fallfilmstripper ST-1 geführt, wo der CO₂-Gehalt der eintretenden Lösung durch auskochendes NH₃ aus der Lösung abgestrippt wird. Die abgestrippten Gase mit der aus der Absorptionskolonne KO-1 1 (Mitteldruckabsorber) rückgeführten Lösung werden in den Carbamatkondensor C geführt und dort kondensiert. Der Druck des Kondensates wird mit der Pumpe P-1 erhöht und dann das Kondensat als Treibmittel für den Ejektor E-1 eingesetzt, um die aus der Melaminanlage stammenden Offgase in den Harnstoffreaktor R-1 fördern zu können.

Eine Lösung mit einem niedrigeren CO₂-Gehalt verläßt nun am Boden den Stripper ST-1 und wird von dort in den Separator entspannt. Im dampfbeheizten Unterteil des Separators, dem Fallfilmzersetzer Z-1, wird nun das meiste verbliebene Carbamat zersetzt, und die NH₃- und CO₂- reichen Gase werden im Mitteldruckkondensator K-1 in einer wäßrigen Carbonatlösung, die über L-1 aus dem Niederdruckteil der Harnstoffanlage stammt, teilweise absorbiert.

Die Gas/Flüssigkeitsmischung aus dem Kondensator K-1 wird in den Mitteldruckabsorber KO-1 geführt und verbliebenes CO₂ und H₂O mit flüssigem NH₃ ausgewaschen. Am Boden des KO-1 wird eine Lösung abgezogen und über die Pumpe P-2 in den Carbamatkondensor C rückgeführt. Das Kopfprodukt des KO-1, reines NH₃-Gas, wird im Kondensator K-2 kondensiert und in den NH₃-Zwischenlagertank gefahren.

### Beispiel 2

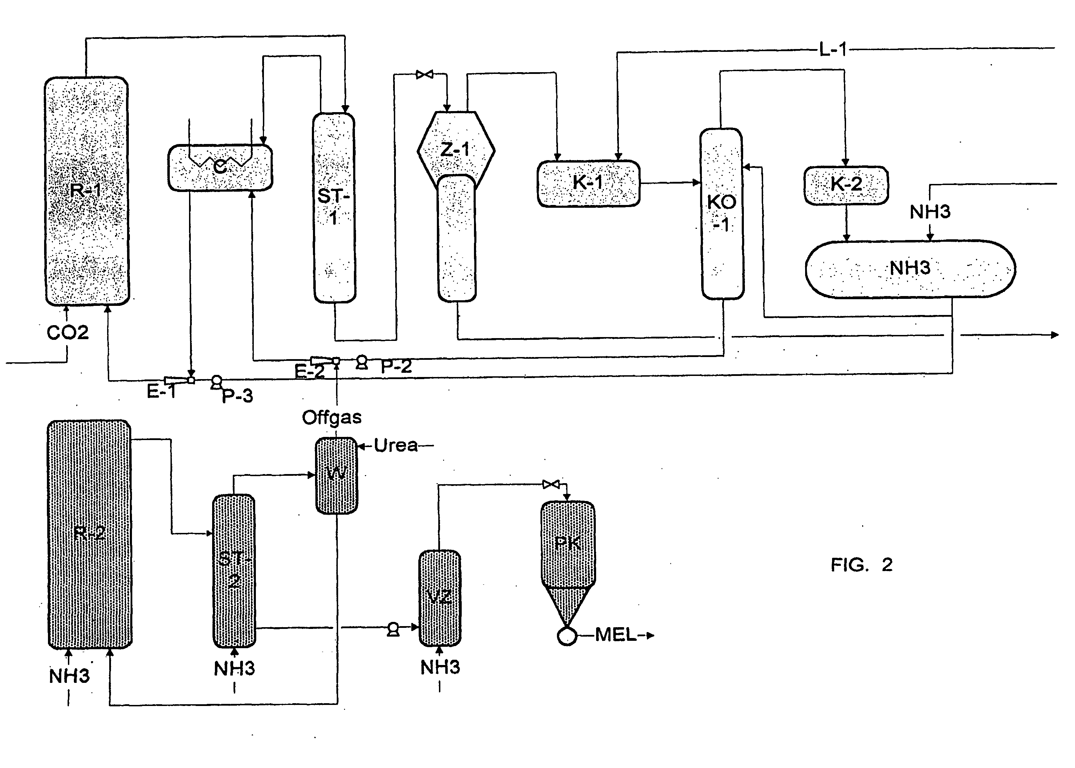

In einer Analge, wie sie schematisch in Fig. 2 dargestellt ist, werden vom Kopf des Harnstoffreaktors R-1 die Reaktionsprodukte zu einem dampfbeheizten Fallfilmstripper ST-1 geführt, wo der CO₂-Gehalt der eintretenden Lösung durch auskochendes NH₃ aus der Lösung abgestrippt wird. Die abgestrippten Gase werden in den Carbamatkondensor C geführt. Der Druck der rückzuführenden Lösung aus der Absorptionskolonne KO-1 wird mit der Pumpe P-2 erhöht und dann als Treibmedium für den Ejektor E-2 eingesetzt, um die aus der Melaminanlage stammenden Offgase in den Carbamatkondensor C fördern zu können. Im Carbamatkondensor werden die Offgase gemeinsam mit den aus dem Stripper ST-1 kommenden Gasen kondensiert.

Eine Lösung mit einem niedrigeren CO₂-Gehalt verläßt nun am Boden den Stripper ST-1 und wird von dort in den Separator entspannt. Im dampfbeheizten Unterteil, dem Fallfilmzersetzer Z-1, wird nun das meiste verbliebene Carbamat zersetzt, und die NH₃- und CO₂- reichen Gase werden im Mitteldruckkondensator K-1 in einer wäßrigen Carbonatlösung, die über L-1 aus dem Niederdruckteil der Harnstoffanlage stammt, teilweise absorbiert.

Die Gas/Flüssigkeitsmischung aus K-1 wird in den Mitteldruckabsorber KO-1 geführt und verbliebenes CO₂ und H₂O mit flüssigem NH₃ ausgewaschen. Am Boden des KO-1 wird eine Lösung abgezogen und über die Pumpe P-2 und Ejektor E-2 in den Carbamatkondensor C rückgeführt. Das Kopfprodukt des KO-1, reines NH₃-Gas, wird im Kondensator K-2 kondensiert und in den NH₃-Zwischenlagertank gefahren.

Aus dem Zwischenlagertank wird flüssiges NH₃ abgezogen, mit der Pumpe P-3 der Druck erhöht und dann als Treibmedium für Ejektor E-1 eingesetzt, um damit das aus dem Carbamatkondensor C kommende Kondensat in den Harnstoffreaktor fördern zu können.

### Beispiel 3

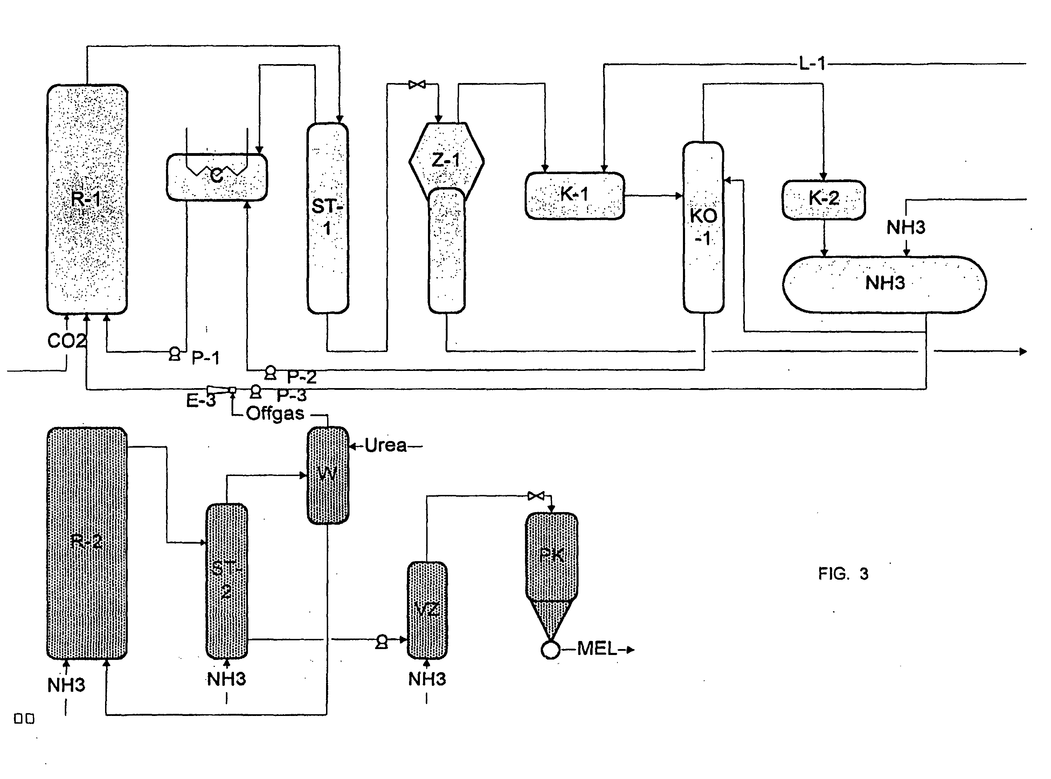

In einer Anlage, wie sie schematisch in Fig. 3 dargestellt ist, werden vom Kopf des Harnstoffreaktors R-1 die Reaktionsprodukte zu einem dampfbeheizten Fallfilmstripper ST-1 geführt, wo der CO₂-Gehalt der eintretenden Lösung durch auskochendes NH₃ aus der Lösung abgestrippt wird. Die abgestrippten Gase mit der aus der Absorptionskolonne KO-1 mit P-2 rückgeführten Lösung werden in den Carbamatkondensor C geführt und dort kondensiert. Der Druck des Kondensates wird nach dem Kondensator mit der Pumpe P-1 erhöht und dann in den Harnstoffreaktor R-1 gefördert.

Eine Lösung mit einem niedrigeren CO₂Gehalt verläßt nun am Boden den Stripper ST-1 und wird von dort in den Separator entspannt. Im dampfbeheizten Unterteil des Separators, dem Fallfilmzersetzer Z-1 wird nun das meiste verbliebene Carbamat zersetzt, und die NH₃- und CO₂- reichen Gase werden im Mitteldruckkondensator K-1 in einer wäßrigen Carbonatlösung, die über L-1 aus dem Niederdruckteil der Harnstoffanlage stammt, teilweise absorbiert.

Die Gas/Flüssigkeitsmischung aus K-1 wird in den Mitteldruckabsorber KO-1 geführt und verbliebenes CO₂ und H₂O mit flüssigem NH3 ausgewaschen. Am Boden des KO-1 wird eine Lösung abgezogen und über die Pumpe P-2 in den Carbamatkondensor C rückgeführt. Das Kopfprodukt des KO-1, reines NH₃-Gas, wird im Kondensator K-2 kondensiert und in den NH₃-Zwischenlagertank gefahren.

Die aus der Melaminanlage stammenden Offgase werden über den Ejektor E-3 in den Hamstoffreaktor gefördert. Das Treibmedium für E-3 ist flüssiges NH₃ aus dem NH₃-Tank, das mit der Pumpe P-3 aufgedrückt wird.

### Beispiel 4

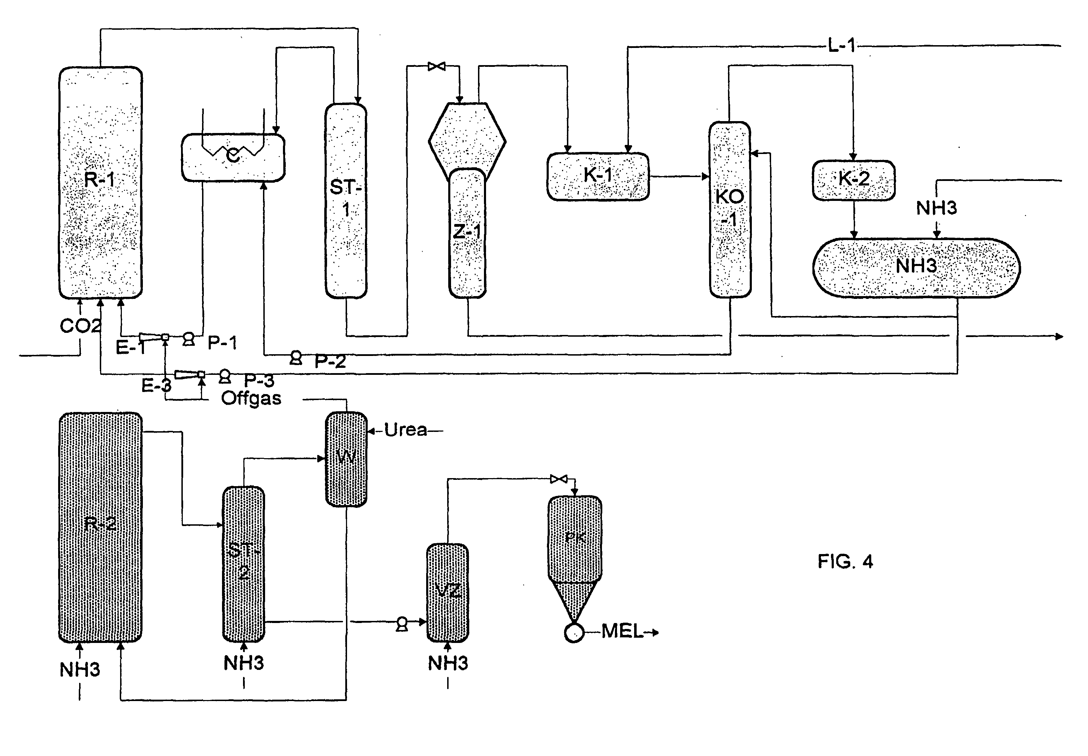

In einer Anlage, wie sie schematisch in Fig. 4 dargestellt ist, werden die Offgase in Kombination der in den Beispielen 1 und 3 beschriebenen Prozeßführung mittels zweier Ejektoren (E-1 und E-3) in den Hamstoffreaktor gefördert. Das Treibmedium für E-1 ist dabei analog zu Beispiel 1 das Kondensat aus dem Carbamatkondensor, das Treibmedium für E-3 analog zu Beispiel 3 flüssiges NH₃ aus dem NH₃-Tank.

## Patentansprüche

1. Verfahren zur Herstellung von Harnstoff, bei dem die aus einer Melaminanlage stammenden, im wesentlichen aus NH₃ und CO₂ bestehenden Gase, mittels eines oder mehrerer Ejektoren direkt in den Hochdruckteil einer Harnstoffanlage eingebracht werden.

2. Verfahren gemäß Anspruch 1, bei dem einer oder mehrere der folgenden Ströme als Treibmedium für die Ejektoren verwendet werden:
a) flüssiges NH₃,
b) gasförmiges NH₃ oder CO₂,
c) im wesentlichen NH₃ und CO₂ enthaltende wäßrige Lösungen.

3. Verfahren gemäß Anspruch 1 oder 2, bei dem die aus der Melaminanlage stammenden Gase einen Druck aufweisen, der im wesentlichen dem Druck im Melaminreaktor entspricht.

4. Verfahren gemäß einem der Ansprüche 1 bis 3, bei dem die aus der Melaminanlage stammenden Gase einen Druck von 50 bis 250 bar und eine Temperatur von 175 bis 250 °C besitzen.

5. Verfahren gemäß Anspruch 4, bei dem die aus der Melaminanlage stammenden Gase einen Druck von 70 bis 200 bar und eine Temperatur von 180 bis 210 °C besitzen.

6. Verfahren gemäß einem der Ansprüche 1 bis 5, bei dem der Druck der Treibmedien höher ist als der Druck im Hochdruckteil der Hamstoffanlage.

7. Verfahren gemäß Anspruch 6, bei dem der Druck der Treibmedien im wesentlichen 1,1 bis 3 mal, bevorzugt 1,3 bis 2,5 mal so hoch ist wie der Druck im Hochdruckteil der Harnstoffanlage.

8. Verfahren gemäß einem der Ansprüche 1 bis 7, bei dem der Druck im Hochdruckteil der Hamstoffanlage im wesentlichen bei 125 bis 350 bar und die Temperatur bei 150 bis 350 °C liegt.

9. Verfahren gemäß Anspruch 8, bei dem der Druck im Hochdruckteil der Harnstoffanlage im wesentlichen bei 140 bis 200 bar und die Temperatur bei 150 bis 210 °C liegt.

## Claims

1. Process for preparing urea in which the gases originating from a melamine plant and consisting essentially of NH₃ and CO₂ are introduced directly into the high-pressure section of a urea plant by means of one or more ejectors.

2. Process according to Claim 1, wherein one or more of the following streams are used as driving medium for the ejectors:
a) liquid NH₃,
b) gaseous NH₃ and CO₂,
c) aqueous solutions containing essentially NH₃ and CO₂.

3. Process according to Claim 11 or 2, wherein the gases originating from the melamine plant have a pressure which corresponds essentially to the pressure in the melamine reactor.

4. Process according to any of Claims 1 to 3, wherein the gases originating from the melamine plant have a pressure of from 50 to 250 bar and a temperature of from 175 to 250°C.

5. Process according to Claim 4, wherein the gases originating from the melamine plant have a pressure of from 70 to 200 bar and a temperature of from 180 to 210°C.

6. Process according to any of Claims 1 to 5, wherein the pressure of the driving media is higher than the pressure in the high-pressure section of the urea plant.

7. Process according to Claim 6, wherein the pressure of the driving media is essentially from 1.1 to 3 times, preferably from 1.3 to 2.5 times, as high as the pressure in the high-pressure section of the urea plant.

8. Process according to any of Claims 1 to 7, wherein the pressure in the high-pressure section of the urea plant is essentially from 125 to 350 bar and the temperature is from 150 to 350°C.

9. Process according to Claim 8, wherein the pressure in the high-pressure section of the urea plant is essentially from 140 to 200 bar and the temperature is from 150 to 210°C.

## Revendications

1. Procédé de fabrication d'urée, lors duquel les gaz provenant d'une installation de mélamine, se composant pour l'essentiel de NH₃ et de CO₂, sont introduits directement à l'aide d'un ou de plusieurs éjecteurs dans la partie haute pression d'une installation d'urée.

2. Procédé selon la revendication 1, lors duquel un ou plusieurs des courants suivants est ou sont utilisés en tant que milieu propulsant pour les éjecteurs :
a) du NH₃ liquide,
b) du NH₃ ou du CO₂ gazeux,
c) des solutions aqueuses contenant pour l'essentiel du NH₃ et du CO₂.

3. Procédé selon la revendication 1 ou 2, lors duquel les gaz provenant de l'installation de mélamine présentent une pression, qui correspond pour l'essentiel à la pression dans le réacteur à mélamine.

4. Procédé selon l'une quelconque des revendications 1 à 3, lors duquel les gaz provenant de l'installation de mélamine possèdent une pression de 50 à 250 bars et une température de 175 à 250°C.

5. Procédé selon la revendication 4, lors duquel les gaz provenant de l'installation de mélamine possèdent une pression de 70 à 200 bars et une température de 180 à 210°C.

6. Procédé selon l'une quelconque des revendications 1 à 5, lors duquel la pression des milieux propulsants est plus élevée que la pression dans la partie haute pression de l'installation d'urée.

7. Procédé selon la revendication 6, lors duquel la pression des milieux propulsants est, pour l'essentiel, de 1,1 à 3 fois, de préférence de 1,3 à 2,5 fois plus élevée que la pression dans la partie haute pression de l'installation d'urée.

8. Procédé selon l'une quelconque des revendications 1 à 7, lors duquel la pression dans la partie haute pression de l'installation d'urée se situe pour l'essentiel à une valeur de 125 à 350 bars et la température se situe à une valeur de 150 à 350°C.

9. Procédé selon la revendication 8, lors duquel la pression dans la partie haute pression de l'installation d'urée se situe pour l'essentiel à une valeur de 140 à 200 bars et la température se situe à une valeur de 150 à 210°C.
